# EUROPÄISCHE PATENTSCHRIFT

(11) **EP 0 469 266 B1**
(45) Veröffentlichungstag und Bekanntmachung des Hinweises auf die Patenterteilung: **17.04.1996**
(21) Anmeldenummer: 91109391.2
(22) Anmeldetag: 07.06.1991
(51) Int. Cl.: A61M 3/02, G05D 16/06

(54) **Vorrichtung zum Spülen der Harnblase**
Bladder flushing device
Inigateur pour la vessie

(30) Priorität: 03.08.1990 DE 4024676
(43) Veröffentlichungstag der Anmeldung: 05.02.1992
(73) Patentinhaber: KNF-NEUBERGER GmbH, D-79112 Freiburg (DE)
(72) Erfinder: Korth, Knut, Dr., W-7802 Merzhausen (DE); Becker, Erich, W-7812 Bad Krozingen (DE)
(74) Vertreter: Schaefer, Konrad, Dipl.-Phys.

(56) Entgegenhaltungen:
- CH-A- 641 964
- US-A- 3 825 029
- US-A- 4 604 089
- US-A- 4 858 619

## Beschreibung

Die Erfindung betrifft eine Vorrichtung der im Oberbegriff des Anspruches 1 genannten Art.

Das Spülen der Harnblase kann unterschiedlichen Zwecken dienen, beispielsweise der postoperativen Behandlung nach Operationen im Blaseninneren bzw. an der Prostata. Als in die Blase verlegtes Instrument dient dann zumeist ein flexibler Katheter, üblicherweise ein Ballonkatheter, der geeignete Spülflüssigkeit dauernd in die Blase spült und aus dieser wieder ablaufen läßt.

Auch bei schneidenden operativen Eingriffen im Inneren der Blase, beispielsweise an der Blasenwand oder an der Prostata, wird gespült. Als in die Blase eingeführtes Instrument dient dann ein starres Endoskop mit einer Optik und einer Schneideinrichtung, üblicherweise einer hochfrequenzbeaufschlagten Schlinge. Die Spülung dient dann zur Schaffung eines freien Gesichtsfeldes, wobei Blut und abgeschnittene Gewebestücke aus dem Gesichtsfeld fortgespült werden.

Das Spülen der Harnblase kann mit unterschiedlichen Instrumenten erfolgen, und zwar, wie bereits erwähnt, mit einem zweikanaligen Katheter oder auch mit einem endoskopischen zweikanaligen Instrument. Es sind aber auch Eingriffe mit zwei getrennt in die Blase zu verlegenden Instrumenten möglich, wobei in der Regel das eine durch die Harnröhre verlegt wird und das andere durch einen suprapubischen Trokar unmittelbar durch die Haut in die Blase verlegt wird. Im letzteren Falle kann beispielsweise einer der beiden zur Spülung erforderlichen Kanäle in einem der beiden Instrumente und der andere in dem anderen der beiden Instrumente angeordnet sein. Es ist aber auch denkbar, beide Kanäle in einem suprapubisch verlegten Instrument vorzusehen.

Beim Spülen ist stets darauf zu achten, daß die Blase einen möglichst gleichmäßigen Füllstand beibehält. Ist der Druck in der Blase zu niedrig, so fällt die Blase zusammen. Insbesondere bei operativen Arbeiten in der Blase kann dies gefährlich werden, wenn dabei die Blasenrückwand plötzlich auf das Instrument zufällt und von diesem unbeabsichtigt verletzt wird.

Andererseits ist ein zu hoher Blasendruck von Nachteil. Wenn gespült wird, liegen stets frisch geschnittene Wunden in der Blase vor, an denen der Blutkreislauf des Patienten zur Blase hin offen ist. Zu hoher Blasendruck führt zum Eindringen von Spülflüssigkeit in den Blutkreislauf des Patienten, dem sogenannten TUR-Syndrom. Das TUR-Syndrom ist gefährlich und muß unter allen Umständen vermieden werden.

Auch bei postoperativer Spülung ist ein zu hoher Blasendruck von Nachteil. Durch Dehnen der Blasenwand können Narben über den frisch operierten Stellen der Blase aufreißen, was die Wundheilung verzögert.

Neben kontinuierlich spülenden Verfahren, bei denen durch eine Zulaufleitung ständig Flüssigkeit zugeführt und durch eine Ablaufleitung Flüssigkeit ständig abgeführt wird, sind auch intermittierende Spülverfahren bekannt, bei denen nur ein Kanal erforderlich ist, der abwechselnd als Zulaufkanal und als Ablaufkanal verwendet wird. Hierbei ist ebenfalls darauf zu achten, daß beim Einleiten von Flüssigkeit ein maximaler Blasendruck nicht überschritten wird, um dann rechtzeitig die Blase wieder zu entleeren und neu zu füllen.

Der angestrebte Druck in der Blase liegt im Bereich weniger Dezimeter Wassersäule. Er wird zumeist eingeregelt mit Ventilen im Zulauf oder im Ablauf. Dabei beobachtet der Operateur die Blasenfüllung mit dem Auge, z.B. durch Betrachtung des Blaseninneren durch die Optik des Endoskopes. Als Einrichtungen zur Regelung des Blasendruckes dienen ihm dabei handbetätigte Ventile.

Als Einrichtungen zur Regelung des Blasendruckes bei Vorrichtungen der eingangs genannten Art sind auch äußerst aufwendige elektronisch gesteuerte Pumpen bekannt, die dafür sorgen, daß Zulaufstrom und Ablaufstrom stets gleich sind. Damit wird zwar das Volumen der Blase konstant gehalten, nicht aber der Druck. Druckerhöhende Einflüsse, die außerhalb des Spülsystems liegen, wie beispielsweise äußerer Druck auf die Blase oder Druckeinflüsse durch die Atmung führen zu Druckerhöhungen, die diese Regeleinrichtung nicht abfangen kann.

Aus der DE-35 40 326 C2 ist eine Vorrichtung bekannt, bei der zusätzlich zu den zum Zu- und Ablauf benötigten Kanälen im Instrument ein Meßkanal vorgesehen ist, der einen Drucksensor mit dem Blasendruck beaufschlagt. Der auf diese Weise gemessene Blaseninnendruck steuert eine Pumpe in der Ablaufleitung, wodurch konstanter Druck in der Blase erzeugt werden kann.

Nachteilig bei dieser Konstruktion ist der erhebliche konstruktive Aufwand, der durch die Druckregelung zusätzlich erforderlich ist. Dieser Aufwand ergibt sich aus der Notwendigkeit, eine Pumpe in der Ablaufleitung vorzusehen, die zudem als Präzisionspumpe ausgebildet sein muß. Ferner muß im Instrument zu den vorhandenen Kanälen ein zusätzlicher Meßkanal vorgesehen werden, was eine völlig Umkonstruktion des Instrumentes erfordert und bei den bekannten, äußerst beschränkten Querschnittsverhältnissen in derartigen Instrumenten erhebliche Unterbringungsprobleme aufwirft.

Eine weitere Spülvorrichtung ist aus der DE-OS 38 05 709 bekannt. Bei ihr ist in der Ablaufleitung ein Überlauf vorgesehen, der den Druck in der Blase stets auf Höhe des statischen Druckes hält, der sich aus der Höhendifferenz zwischen dein Überlauf und der Blase ergibt. Allerdings erfolgt hier die Erfassung des Blasendruckes in der Ablaufleitung, die prinzipbedingt stets verstopfungsgefährdet ist. Bei Operationen in der Blase freigesetzte Gewebestückchen können die Ablaufleitung verstopfen. Daher ist diese Konstruktion nur möglich unter Verwendung eines suprapubischen Trokars als Ablaufleitung, bei dem geeignet große Querschnitte gewählt werden können, die die Verstopfungsgefahr ausschließen. Bei Instrumenten, die sowohl die Zulaufleitung als auch die Ablaufleitung aufnehmen müssen und somit nur sehr enge Querschnitte zur Verfügung stellen können, ist diese Konstruktion nicht einsetzbar. Sie hat daher den Nachteil eines zusätzlichen operativen Eingriffes und ist schwierig handhabbar.

Eine gattungsgemäße Spülvorrichtung ist aus der CH-A-641 964 bekannt. Bei dieser Konstruktion ist in der Zulaufleitung eine Einrichtung zur Regelung des Blasendruckes vorgesehen, die diesen stets konstant hält. Die Probleme der eingangs genannten Art mit schwankenden Druckverhältnissen in der Blase werden dadurch beseitigt. Bei dieser Konstruktion ist die Regelungseinrichtung als höhenfest angeordnete Schwimmerkammer vorgesehen, bei der der Schwimmer den Zufluß in die Kammer auf ein wählbares Niveau regelt. Die Schwimmerkammer ist in geeigneter Höhe über der Blase vorzusehen und hält dann auf Höhe des Schwimmers eine Wassersäule zwischen dem Schwimmer und der Blase auf konstanter Höhe, welche den gewünschten konstanten Druck in der Blase ergibt.

Bei dieser Konstruktion ist zwar innerhalb der Schwimmerkammer ein, Höhenverstellbereich und somit ein Druckverstellbereich vorgesehen, jedoch ist die sich ergebende Wassersäule von der Grundhöheneinstellung der Schwimmerkammer abhängig. Die Schwimmerkammer muß daher stets auf erforderlicher Höhe, und zwar auf jeden Fall oberhalb der Blase angeordnet werden, da sonst der erforderliche Druck in der Blase nicht erreicht werden kann. Daraus ergeben sich Nachteile hinsichtlich der Anordnung der ohnehin sperrigen Schwimmerkammer auf dem regelmäßig mit sonstigen Geräten bereits überlasteten Operationsgebiet. Die Schwimmerkammer muß außerdem stets in einer Höhe von einigen Dezimetern über der Blase des Patienten angeordnet sein, also gerade in dem Bereich, in dem das Operationspersonal tätig ist. Daraus ergeben sich Störungen des Operationsbetriebes.

Weitere Nachteile dieser Konstruktion ergeben sich aus den mangelhaften Sterilitätsverhältnissen. Bei heutiger hochsteriler Arbeitsweise wird als Zulaufreservoir für die Spülflüssigkeit stets entweder ein Sterilfilter oder ein luftfrei geschlossener Sterilbeutel verwendet. Aus diesem muß steril bis zur Blase gefördert werden.

Bei der bekannten Konstruktion ist aber zwingend in dem Schwimmerbehälter oberhalb des Schwimmers ein Luftvolumen erforderlich, das auch im Austausch mit der umgebenden Atmosphäre stehen muß, da sonst der Schwimmer nicht ordnungsgemäß arbeiten kann. Dieses Luftvolumen muß der Schwimmerkammer von außen zugeführt werden, was zu Sterilitätsproblemen führt, oder in aufwendiger Weise gefiltert werden. Hierdurch ergeben sich nicht vermeidbare Nachteile dieser Konstruktion, die zumindest die Wartungs- und Betriebskosten wesentlich in die Höhe treiben.

Die Aufgabe der vorliegenden Erfindung besteht darin, eine Vorrichtung der eingangs genannten Art zu schaffen, die bei kostengünstiger Anwendbarkeit die automatische Blasendruckregelung bei einfacher Handhabbarkeit und hoher Sicherheit ermöglicht.

Diese Aufgabe wird erfindungsgemäß mit den Merkmalen des Kennzeichnungsteiles des Anspruches 1 gelöst.

Die erfindungsgemäße Vorrichtung weist einen Druckregler in der Zulaufleitung auf. Dies hat zunächst den prinzipbedingten Vorteil, daß die Zulaufleitung durch einströmende Spülflüssigkeit stets freigehalten wird, also nicht verstopfen kann, so daß der Sensor des Druckreglers stets den Druck in der Blase erfaßt. Der vom Sensor erfaßte Druck stimmt im wesentlichen mit dem Druck in der Blase überein, da bei den geringen erforderlichen Spülströmen in der Größenordnung einiger ml/Min. auch bei engen Kanälen der dynamische Druckabfall vernachlässigbar bleibt. Einzubeziehen ist allerdings der statische Druck, der sich aus dem Höhenunterschied zwischen dem Druckregler und der Blase ergibt. Daher ist der Druckregler auf geeignete Weise höhenfest vorzusehen. Der Druckregler kann als einfaches, den Spülstrom beeinflussendes Ventil vorgesehen sein, das von einem Drucksensor gesteuert wird, welcher blasenseitig vom Ventil vorgesehen ist. Ein solcher Druckregler kann, wie im folgenden gezeigt wird, sehr einfach wartungsfrei ausgebildet sein und bei entsprechenden Einstellmöglichkeiten eine einfache Druckeinstellung auf die gewünschte Höhe ermöglichen. Der Druckregler arbeitet völlig unabhängig von irgendwelchen Verstopfungen in der Abflußleitung. Wenn diese völlig verstopft ist, so schließt er das Ventil und hält immer noch den Druck in der Blase auf der vorgegebenen Höhe. Insbesondere hält der Druckregler den Druck in der Blase unabhängig von der Höhe des Spülstromes konstant. Die Höhe des Spülstromes kann auf andere Weise, beispielsweise durch geeignete Abflußregulierung, gewählt werden. Der Druckregler eignet sich zum Einsatz bei allen bekannten Spülmethoden, also insbesondere bei kontinuierlicher Dauerspülung oder auch bei intermittierendem Spülen. Der Druckregler ist als Membrandruckregler wegen des möglichen einfachen mechanischen Aufbaus kostengünstig herstellbar und eignet sich zur langfristig sicheren feinfühligen Druckregelung. Die Federbelastung der Membran kann durch deren Eigenfederwirkung oder mittels Zusatzfeder erreicht werden.

Vorteilhaft sind dabei die Merkmale des Anspruches 2 vorgesehen. Auf diese Weise kann der Druckregler als Zusatzgerät bei bekanntem Instrumentarium nachgerüstet, beispielsweise in der zum Instrument führenden Schlauchleitung zwischengeschaltet werden.

Vorteilhaft sind die Merkmale des Anspruches 4 vorgesehen. Die Konusfläche des Ventiltellers ergibt mit dem Rand der Ventilöffnung eine kreisförmige Liniendichtung hoher Qualität Außerdem wirkt die Konusfläche bei jedem Schließen des Ventiles selbstzentrierend, so daß der Ventilkörper und die Membran durch das Ventilspiel stets selbst zentriert werden. Zusätzliche Zentriereinrichtungen sind daher nicht erforderlich.

Weiterhin vorteilhaft sind die Merkmale des Anspruches 5 vorgesehen. Mit der Feder kann ein höherer Regeldruck eingestellt werden bzw. ist auch die Verwendung einer nicht selbstrückstellenden Membran möglich. Die Feder ist im Luftraum angeordnet, braucht dort also nicht sterilisiert zu werden und ist nicht dem Angriff der Spülflüssigkeit ausgesetzt.

Dabei sind vorteilhaft die Merkmale des Anspruches 6 vorgesehen. Auf diese Weise läßt sich der Blasendruck feinfühlig mittels der Stelleinrichtung verstellen.

Weiterhin vorteilhaft sind die Merkmale des Anspruches 7 vorgesehen. Mit der Betätigungseinrichtung kann von Hand das Ventil geöffnet werden, was z.B. voll Vorteil ist beim ersten Befüllen des noch leeren Ventiles, um störende Luft auszuspülen.

Weiterhin vorteilhaft sind die Merkmale des Anspruches 8 vorgesehen. Auf diese Weise wird der Druckregler vereinfacht und verbilligt und mit gut sterilisierbaren Flächen versehen bzw. kann als Einmalartikel ausgebildet sein, der nach einmaligem Gebrauch weggeworfen wird. Die Sterilisierung des Druckreglers ist vor jedem Gebrauch erforderlich, da er in der Zulaufleitung sitzt.

Weiterhin vorteilhaft sind die Merkmale des Anspruches 9 vorgesehen. Auf diese Weise läßt sich unabhängig von der Überwachung des Blasendruckes der Spülflüssigkeitsdurchfluß überwachen. Dies ist bei der erfindungsgemäßen Blasendruckregelung besonders wichtig, da diese verhindert, daß Spülflußänderungen sich deutlich bemerkbar machen. Eine gesonderte Anzeige des Durchflusses ist daher von Vorteil.

In den Zeichnungen ist die Erfindung beispielsweise und schematisch dargestellt. Es zeigen:
- Fig. 1: eine schematische Darstellung einer erfindungsgemäßen Spülvorrichtung mit einem Resektoskop in der im Schnitt dargestellten Blase und mit dem Druckregler der Fig. 2 und
- Fig. 2: einen Schnitt nach Linie 2 - 2 in Fig. 1 durch den Druckregler.

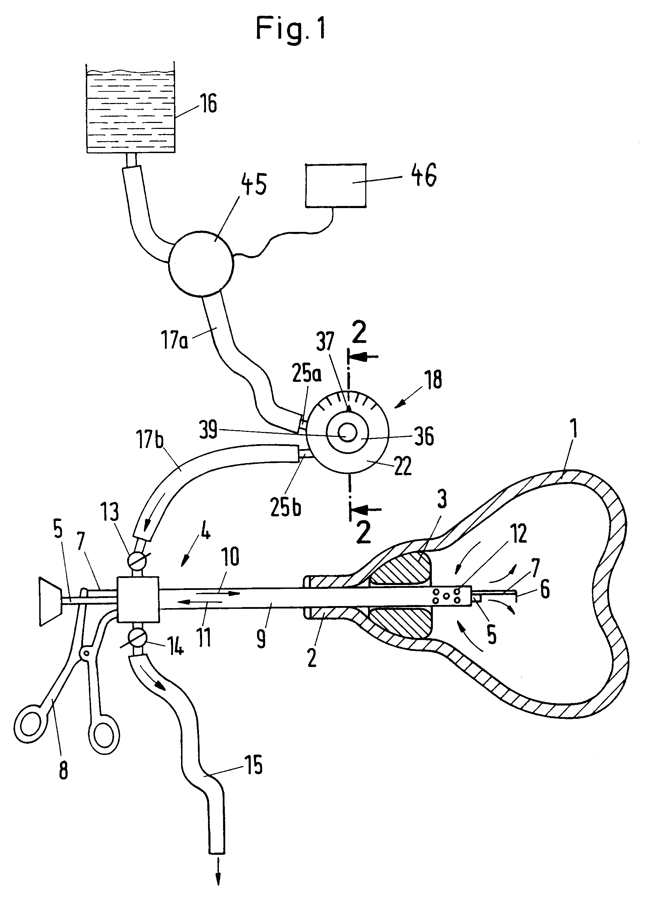

Fig. 1 zeigt eine Blase 1 im Schnitt, und zwar die Harnblase eines Menschen. Es ist auch der Übergang zur Harnröhre 2 dargestellt, an der die Prostata 3 sitzt.

In die Blase ist ein Resektoskop 4 eingeführt, ein in der Urologie häufig verwendetes Endoskop mit einer Optik 5 und einer hochfrequenzbeaufschlagten Schneidschlinge 6 an einem Schlingenträger 7, dessen proximales Ende über einen Scherengriff 8 in Axialrichtung betätigbar ist.

Optik 5 und Schlingenträger 7 sind in einem umgebenden Schaft 9 angeordnet, der auf geeignete Weise in Längsrichtung in einen Zulaufkanal 10 und einen Ablaufkanal 11 geteilt ist, welche in der Fig. 1 schematisch mit Pfeilen dargestellt sind. Wie die Figur zeigt, ist der Zulaufkanal 10 am distalen Ende des Schaftes 9 in axialer Richtung offen, so daß dort in Pfeilrichtung Spülflüssigkeit in die Blase austritt. Der Ablaufkanal 11 mündet am distalen Ende des Schaftes 9 in äußeren Löchern 12, in die die Spülflüssigkeit in der dargestellten Pfeilrichtung aus der Blase eintritt.

In üblicher Weise ist am proximalen Ende des Schaftes 9 ein an den Zulaufkanal 10 angeschlossener Zulaufhahn 13 und ein an den Ablaufkanal 11 angeschlossener Ablaufhahn 14 angeschlossen. Vom Ablaufhahn 14 geht ein Ablaufschlauch 15 zu einem nicht dargestellten Abfluß.

Dem Zulaufhahn 13 strömt Spülflüssigkeit von einem Reservoir 16 zu, das üblicherweise an erhöhter Stelle aufgehängt wird und auf diese Weise den erforderlichen Spüldruck liefert. Das Reservoir 16 ist mit dem Zulaufhahn 13 über einen Zulaufschlauch 17a, 17b verbunden, wobei zwischen dessen beiden Teilen ein Druckregler 18 zwischengeschaltet ist.

Mit dem dargestellten Resektoskop 4 kann der Operateur durch die Optik 5 das Blaseninnere übersehen und nach Bringen des Instrumentes in eine gewünschte Operationsstellung durch Vor- und Rückwärtsbewegung der Schneidschlinge 6 Schnitte vornehmen, mit denen beispielsweise Karzinome von der die Blase auskleidenden Schleimhaut entfernt werden oder die Prostata 3 von innen abgehobelt wird.

Dabei entstehen starke Blutungen, und es schwimmen Gewebeteilchen in der Blase herum. Ein steter Spülstrom in Richtung der in der Blase dargestellten Pfeile sorgt für ein Freihalten des Gesichtsfeldes der Optik 5.

Dabei ist dafür Sorge zu tragen, daß der Innendruck der Blase konstant bleibt, die Blase also stets einen Entfaltungsgrad beibehält, der beispielsweise etwa der dargestellten Form entspricht. Dabei ist die Blase weit genug entfaltet, daß alle ihre Teile zugänglich sind, und es besteht genug Arbeitsraum in der Blase, um operieren zu können. Der Druck ist aber noch nicht hoch genug, um an frisch operierten, blutenden Stellen ein Eindrücken der Spülflüssigkeit in die Blutgefäße zu ermöglichen.

Wichtig ist dabei, daß sich der Druck nicht plötzlich ändert. Würde der Druck stark abfallen, so könnte die rückwärtige Blasenwand auf das Instrument zu gedrückt werden und ungewollten Kontakt mit der Schneidschlinge 6 bekommen, was zu Verletzungen oder sogar zum Durchschneiden der Blasenwand führen kann.

Der dargestellte Druckregler 18 sorgt daher für eine hochgenaue Einhaltung des Blasendruckes.

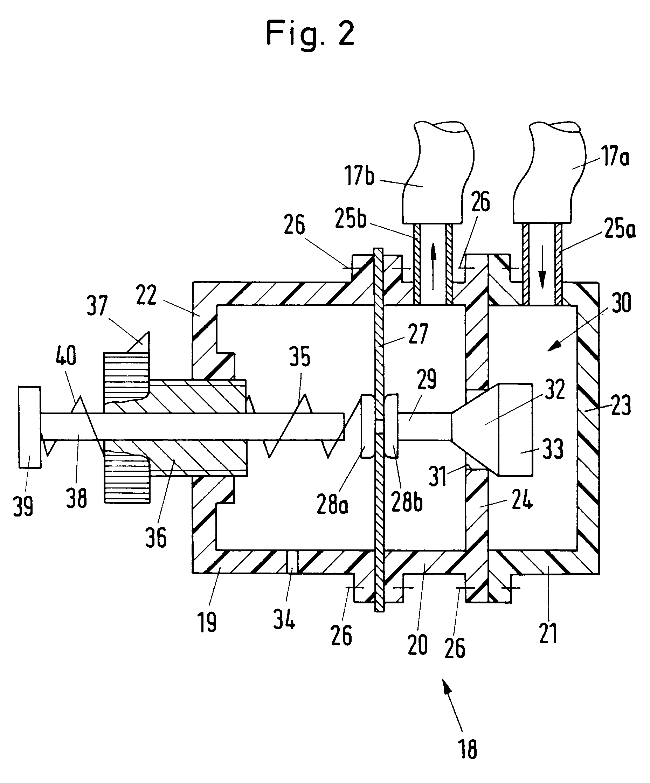

Fig. 2 zeigt den Druckregler 18 im Schnitt der Linie 2 - 2 in Fig. 1. Der Druckregler ist im dargestellten Ausführungsbeispiel, wie die Fig. 1 zeigt, in der Achsansicht kreisförmig ausgebildet. Er besitzt ein Luftkammergehäuse 19, ein Ablaufkammergehäuse 20 und ein Zulaufkammergehäuse 21. Alle Gehäuse weisen zylindrische Gehäuseteile gleichen Durchmessers auf sowie jeweils eine Querwand. Dabei bildet die Querwand des Luftkammergehäuses 19 die bedienungsseitige Stirnwand 22 des Druckreglers und die Querwand des Zulaufkammergehäuses 21 dessen Rückwand 23. Die Querwand des Ablaufkammergehäuses 20 bildet eine Trennwand 24 zwischen der Ablaufkammer und der Zulaufkammer. An die Ablaufkammer im Ablaufkammergehäuse 20 ist der ablaufende Schlauch 17b angeschlossen. An die Zulaufkammer im Zulaufkammergehäuse 21 ist der vom Reservoir 16 kommende Zulaufschlauch 17a angeschlossen, und zwar über jeweils geeignete Anschlußstutzen 25a und 25b.

Im dargestellten Ausführungsbeispiel liegen die Gehäuseteile 19, 20 und 21 jeweils mit Flanschen abgedichtet aneinander, die, wie schematisch mit Strichen 26 angedeutet, verschraubt, verklebt, verklammert oder sonstig verbunden sein können.

An der Flanschverbindung zwischen dem Luftkammergehäuse 19 und dem Ablaufkammergehäuse 20 ist eine die beiden Kammern trennende elastische Membran 27 eingeklemmt. Mittig ist an dieser mit geeigneten Haltetellern 28a und 28b die Ventilstange 29 eines Ventilkörpers 30 befestigt, welcher eine Ventilöffnung 31 in der Trennwand 24 durchsetzt und in der Zulaufkammer im Zulaufkammergehäuse 21 einen Ventilteller 33 ausbildet, dessen zur Membran 27 hin gelegene Fläche als Konusfläche 32 ausgebildet ist, welche am einen Ende von engerem und am anderen Ende von größerem Querschnitt ist als die Öffnungsweite der kreisförmigen Ventilöffnung 31.

Die Luftkammer im Luftkammergehäuse 19 ist mit einer Öffnung 34 nach außen zur Atmosphäre hin offen. In der Luftkammer ist eine Schraubenfeder 35 vorgesehen, die in der Achse der Ventilstange 29 angeordnet ist und sich einerseits auf der Membran 27 abstützt und andererseits an einer Stellschraube 36, die in einem Gewinde der Stirnwand 22 in Achsrichtung der Ventilstange 29 eingeschraubt ist. Durch Verstellen der Stellschraube 36 kann die Rückstellkraft für die Membran 27 feinfühlig eingestellt werden. Die eingestellte Position kann mit einer Nase 37 auf der Stellschraube 36 gegenüber einer Skala auf der Stirnwand 22 (siehe Fig. 1) überwacht werden.

Die Stellschraube 36 wird in Achsrichtung von einem Stößel 38 durchsetzt, der mit einem Druckknopf 39 entgegen einer Schraubenfeder 40, die sich zwischen dem Druckknopf 39 und der Stellschraube 36 abstützt, eingedrückt werden kann, um nach Überwindung eines Spiels (wie in Fig. 2 dargestellt) die Membran 27 in Öffnungsrichtung des Ventiles 31, 32 (also nach rechts gemäß Fig. 2) zu bewegen.

Der dargestellte Druckregler 18 arbeitet wie folgt:
Er wird zunächst, wie in Fig. 1 dargestellt, angeschlossen. Es strömt nun Spülflüssigkeit aus dem Reservoir 16 in den Druckregler, dessen Ventil unter dem Druck der Feder 35 offen ist. Die Spülflüssigkeit füllt zunächst die Kammern des Druckreglers in den Gehäuseteilen 20 und 21. Dabei wird vorteilhaft der Stößel 38 mit dem Druckknopf 39 kurzzeitig eingedrückt und das Ventil in Öffnungsstellung gehalten, damit Luftblasen in den Kammern rasch ausgespült werden. Die Spülflüssigkeit strömt weiter durch den Schlauch 17b und den Zulaufkanal 10 in die Blase, um aus dieser, wie in Fig. 1 dargestellt, abzuströmen. Das Ventil im Druckregler 18 wird so lange offengehalten, bis ein bestimmter Druck in der Blase erreicht ist. Dann drückt die Spülflüssigkeit mit wachsender Kraft die Membran 27 gegen die Feder 35, so daß die Membran 27 auslenkt und das durch die Ventilöffnung 31 und die Konusfläche 32 gebildete Ventil schließt. Dabei wird aufgrund der Linienberührung ein guter Ventilschluß erreicht, und es erfolgt eine Zentrierung des Ventiltellers 33 und somit auch der Membran, sollte diese seitlich ausgelenkt sein.

Nun ist das Ventil geschlossen, und es strömt keine Spülflüssigkeit mehr in die Blase. Bei weiterem Ablauf durch die Ablaufleitung 11, 15 sinkt der Druck in der Blase und folglich auch die die Membran 27 auslenkende Kraft. Die Feder 35 überwiegt in ihrer Kraft wieder die die Membran 27 auslenkende Druckkraft der Flüssigkeit und öffnet das Ventil, so daß wieder Flüssigkeit zuströmt und den Blasendruck erhöht. Das Ventilregelspiel beginnt von neuem.

Der Operateur beobachtet zu Beginn der Spülung mittels der Optik 5 des Resektoskopes 4 das Blaseninnere und verstellt durch Verdrehen der Stellschraube 36 den Blasendruck, bis er das Blaseninnere in der gewünschten Öffnungsstellung sieht. Er kann auch mit einer vorgewählten Druckstellung arbeiten, die er an der Stellschraube 36 vorwählt aufgrund von Erfahrungen früherer Operationen.

Der Druckregler 18 muß in konstanter Höhe über der Blase 1 vorgesehen sein. Dazu kann ein geeignetes Gestell vorgesehen werden oder er wird einfach auf dem Bauch des Patienten aufgelegt.

Die Erfindung kann auch bei postoperativer Spülung eingesetzt werden. Anstelle des Resektoskopes 4 liegt dann in der Blase ein Spülkatheter, beispielsweise ein Ballonkatheter, mit Zulauf- und Ablaufkanal, entsprechend angeschlossen an die Schläuche 17b und 15. Mit einer solchen Anordnung kann langfristig risikoarm gespült werden, ohne daß dauernde Überwachung des Blasendruckes durch das Personal erforderlich ist. Es kann im Druckregler ein Alarmgeber vorgesehen sein, der bei zu starker Auslenkung der Membran 27 anspricht und das Bedienungspersonal ruft.

Der dargestellte Druckregler 18 kann auf vielfältige Weise im Rahmen der Erfindung variiert werden. Beispielsweise ist er nach geeigneter Miniaturisierung in ein Instrument einbaubar, beispielsweise in das in Fig. 1 dargestellte Resektoskop 4 oder in den alternativ besprochenen Ballonkatheter.

Gegenüber der konstruktiven Ausbildung der Fig. 2 kann das Innenleben des Druckreglers weitgehend variiert werden. So kann die mechanische Übertragung zwischen Membran 27 und Ventilteller 33 auf andere Weise erfolgen, beispielsweise über Kniehebelkonstruktionen od.dgl. Die Einstellmöglichkeiten für die Federkraft der Membran 27 können auf andere Weise gelöst werden als im dargestellten Falle. Beispielsweise kann die Luftkammer im Luftkammergehäuse 19 abgedichtet und mit einer Gasdruckquelle verbunden sein, welche einen geregelten Überdruck auf der Gasseite der Membran 27 erzeugt und auf diese Weise den Blasendruck einstellt.

Das Innere der Gehäuseteile 20 und 21 muß vor Gebrauch vollständig sterilisiert werden, da diese Innenflächen mit dem Spülwasser in Berührung kommen. Der Druckregler kann zu diesem Zweck sterilisierbar als wiederverwendbares Gerät ausgebildet sein und beispielsweise im ganzen autoklaviert werden oder nur mittels Durchströmung seiner flüssigkeitsgefüllten Kammern mit Desinfektionsflüssigkeit, die an den Stutzen 25a und 25b angeschlossen wird. Dabei wird zum Sterilisieren dauernd der Stößel 38 eingedrückt gehalten, um das Ventil in geöffneter Stellung zu halten.

Aufgrund seiner einfachen und kostengünstigen Konstruktion kann der gesamte Druckregler auch in Kunststoffausführung seiner Gehäuseteile als Einmalartikel ausgebildet werden, der nach einmaligem Gebrauch weggeworfen wird.

Anstelle der Membran 27 kann auch ein anderer Typ von Drucksensor verwendet werden, beispielsweise ein piezoelektrischer Drucksensor, der das Ventil beispielsweise elektromotorisch steuert.

Wie Fig. 1 zeigt, ist im dargestellten Ausführungsbeispiel in dem Zulaufschlauch 17a eine Durchflußmeßeinrichtung 45 angeordnet, die die Strömungsgeschwindigkeit überwacht und an eine Einrichtung 46 angeschlossen ist, die beispielsweise derart ausgebildet ist, daß sie bei Unterschreiten einer bestimmten Strömungsgeschwindigkeit Alarm gibt.

Hiermit kann der Operateur sofort alarmiert werden. Dafür gibt es mehrere Gründe. Es kann beispielsweise das Reservoir 16 leer sein. Es kann einer der Schläuche 17a, 17b oder 15 durch einen Knick verschlossen sein. Hauptgrund ist zumeist aber ein Verstopfen des Ablaufkanales 11, der durch Operationsreste verschlossen wird, die beispielsweise die Löcher 12 am distalen Ende des Instrumentes verstopfen.

Der Operateur würde ein Versiegen des Spülstromes sonst nicht oder nur viel zu spät merken, da die erfindungsgemäße Druckregelung den Druck in der Blase 1 konstant hält, sich für den Operateur also unabhängig von der Größe des Spülstromes der Blasenzustand nicht ändert.

Als Durchflußmeßeinrichtung 45 können geeignete Einrichtungen verwendet werden, die im Stand der Technik bekannt sind.

Die Durchflußmeßeinrichtung 45 kann auch an anderer als der dargestellten Stelle vorgesehen sein, beispielsweise in den Druckregler integriert. Sie kann jedoch auch im Ablaufschlauch 15 vorgesehen sein oder in das Instrument 4 integriert sein, also im Zulaufkanal 10 oder im Ablaufkanal 11.

Anhand der Fig. 1 wurde eine Anwendung des erfindungsgemäßen Prinzipes bei einem Dauerspülresektoskop 9 geschildert, bei dem in demselben Instrument sowohl der Zulaufkanal 10 als auch der Ablaufkanal 11 vorgesehen sind. Die erfindungsgemäße Druckregelung kann aber auch bei Instrumenten mit intermittierender Spülung eingesetzt werden, die nur einen Kanal aufweisen, durch den sowohl der Zulauf als auch der Ablauf erfolgt und bei denen abwechselnd die Blase gefüllt und wieder entleert wird. Auch hier kann die Begrenzung des maximalen Druckes mit großem Vorteil eingesetzt werden.

Weiterhin ist eine Anwendung möglich dann, wenn der Zulauf und der Ablauf über getrennte Instrumente erfolgt, wobei beispielsweise ein Resektoskop ähnlich dem in Fig. 1 dargestellten Instrument 1 durch die Harnröhre in die Blase verlegt ist und ein suprapubischer Katheter von außen durch die Bauchwand in die Blase durch einen Stichkanal gelegt ist. Diese Methode setzt sich neuerdings zunehmend durch und bietet den Vorteil, in zwei Instrumenten Kanäle großen Querschnittes zur Verfügung stellen zu können. Auch hier kann der Zulauf erfindungsgemäß mit einer Druckregelung versehen sein.

Schließlich läßt sich die Erfindung von Vorteil einsetzen bei Kathetern, die postoperativ, also nach einer Operation für einige Zeit zur Spülung der Blase eingesetzt werden. Auch hierbei ist die erfindungsgemäße Druckregelung von Vorteil. Die Anschlüsse können in gleicher Weise vorgesehen sein, wie in Fig. 1 anhand des Instrumentes 9 gezeigt.

## Patentansprüche

1. Vorrichtung zum Spülen der Harnblase (1), mit wenigstens einem von außen bis in die Blase zu verlegenden Instrument (4) und einer Zulauf- (17a, 17b, 10) sowie einer Ablaufleitung (11, 15), die jeweils teilweise innerhalb des jeweiligen Instrumentes, im Bereich dessen distaler Spitze mündend angeordnet sind und teilweise, am proximalen Ende des Instrumentes abgehend, außerhalb des Instrumentes, von einem unter Druck Spülflüssigkeit liefernden Rerservoir (16) kommend bzw. zu einem Abfluß führend angeordnet sind, und mit einer Einrichtung zur Regelung des Blasendruckes, die einen Druckregler (18) aufweist, der höhenfest in einem die Zulaufleitung (17a, 17b, 10) bildenden Meßkanal angeordnet ist und ein den Durchflußquerschnitt veränderndes Ventil (31, 32) aufweist, das von einein auf der zur Blase (1) hin gelegenen Seite des Ventils den Druck in der Zulaufleitung (17b, 10) ermittelnden Drucksensor gesteuert ist, **dadurch gekennzeichnet**, daß der Drucksensor als auf einer Seite von der Spülflüssigkeit und auf der anderen Seite von Umgebungsluft beaufschlagte federbelastete Membran (27) ausgebildet ist, die höhenfest angeordnet ist und deren Auslenkung auf mechanischem Wege den Ventilkörper (30) des Ventils betätigt.

2. Vorrichtung nach Anspruch 1, **dadurch gekennzeichnet**, daß der Druckregler (18) im außerhalb des Instrumentes gelegenen Teil (17a, 17b) der Zulaufleitung (17a, 17b, 10) angeordnet ist.

3. Vorrichtung nach Anspruch 1, **dadurch gekennzeichnet,** daß eine Ventilstange (29) des Ventilkörpers (3) an der zur Spülflüssigkeit hin liegenden Seite der Membran (27) befestigt ist und, im wesentlichen senkrecht zur Membran verlaufend, eine Ventilöffnung (31) in einer im wesentlichen parallel zur Membran verlaufenden Trennwand (24) durchsetzt, an deren Rückseite sie einen Ventilteller (33) trägt, wobei die Trennwand zwischen einer an das Reservoir (16) angeschlossenen Kammer und einer an die Blase (1) ausgeschlossenen, zur Membran (27) hin gelegenen Kammer vorgesehen ist.

4. Vorrichtung nach Anspruch 3, **dadurch gekennzeichnet**, daß die Ventilöffnung (31) kreisförmig ist und die zur Membran (27) hin liegende Fläche des Ventiltellers (33) zum Schaft (29) hin sich konisch verjüngend ausgebildet ist, wobei der engste Querschnitt der Konusfläche (32) kleiner und ihr größter Querschnitt größer ist als die Ventilöffnung (31).

5. Vorrichtung nach einem der Ansprüche 1 bis 4, **dadurch gekennzeichnet**, daß die Membran (27) luftseitig mit einer Feder (35) abgestützt ist.

6. Vorrichtung nach Anspruch 5, **dadurch gekennzeichnet**, daß die Feder (35) mit einer von außen verstellbaren Stelleinrichtung (36) in ihrer Federkraft verstellbar ist.

7. Vorrichtung nach einem der Ansprüche 1 bis 6, **dadurch gekennzeichnet**, daß eine Betätigungseinrichtung vorgesehen ist, die nach Überwindung eines Spieles auf den Ventilkörper (30) in Öffnungsrichtung einwirkt.

8. Vorrichtung nach einem der Ansprüche 1 bis 7, **dadurch gekennzeichnet**, daß die Gehäuseteile (19, 20, 21) des Druckreglers (18) aus Kunststoff ausgebildet sind.

9. Vorrichtung nach einem der vorhergehenden Ansprüche, **dadurch gekennzeichnet**, daß in der Zulaufleitung (17a, 17b, 10) oder in der Ablaufleitung (15, 11) eine Durchflußmeßeinrichtung (45) vorgesehen ist.

## Claims

1. Apparatus for flushing the bladder (1) with at least one instrument (4) to be introduced into the bladder from the exterior and a supply line (17a, 17b, 10) and a drain line (11, 15), which are each arranged partially within the respective instrument, opening out in the region of its distal tip, and are arranged partially outside the instrument, leaving the instrument at the proximal end, coming from a reservoir (16) supplying flushing liquid under pressure and leading to a drain, respectively, and with a device for regulating the bladder pressure, which has a pressure regulator (18), which is vertically fixedly arranged in a measuring passage constituting the supply line (17a, 17b, 10) and has a valve (31, 32), which alters the flow cross-section and is controlled by a pressure sensor which determines the pressure in the supply line (17b, 10) on the side of the valve closest to the bladder (1), characterised in that the pressure sensor is constructed as a spring-loaded membrane (27), which is acted on by the flushing liquid on one side and by atmospheric air on the other side and which is vertically fixedly disposed and whose deflection actuates the valve body (30) of the valve mechanically.

2. Apparatus as claimed in Claim 1, characterised in that the pressure regulator (18) is arranged in the portion (17a, 17b) of the supply line (17a, 17b, 10) situated outside the instrument.

3. Apparatus as claimed in Claim 1, characterised in that a valve rod (29) of the valve body (3) is secured on the side of the membrane (27) directed towards the flushing liquid and, extending substantially perpendicular to the membrane, passes through a valve opening (31) in a partition wall (24), which extends substantially parallel to the membrane and on whose rear side it carries a valve plate (33), whereby the partition wall is provided between a chamber connected to the reservoir (16) and a chamber connected to the bladder (1) and directed towards the membrane (27).

4. Apparatus as claimed in Claim 3, characterised in that the valve opening (31) is circular and the surface of the valve plate (33) directed towards the membrane (27) is of conically tapering construction towards the shaft (29), whereby the smallest cross-section of the conical surface (32) is smaller and its largest cross-section is larger than the valve opening (31).

5. Apparatus as claimed in one of Claims 1 to 4, characterised in that the membrane (27) is acted upon on the air side by a spring (35).

6. Apparatus as claimed in Claim 5, characterised in that the spring force of the spring (35) is adjustable with an adjustment device (36) which may be adjusted from the exterior.

7. Apparatus as claimed in one of Claims 1 to 6, characterised in that an actuating device is provided which, after overcoming a clearance, acts on the valve body (30) in the opening direction.

8. Apparatus as claimed in one of Claims 1 to 7, characterised in that the housing members (19, 20, 21) of the pressure regulator (18) are made of plastic.

9. Apparatus as claimed in one of the preceding claims, characterised in that a flow measuring device (45) is provided in the supply line (17a, 17b, 10) or in the drain line (15, 11).

## Revendications

1. Dispositif d'irrigation de la vessie (1), ce dispositif comportant au moins un instrument (4) devant être mis en place dans la vessie à partir de l'extérieur et un conduit d'alimentation (17a, 17b, 10) ainsi qu'un conduit d'évacuation (11, 15), qui sont disposés chacun partiellement à l'intérieur de l'instrument considéré en débouchant dans la zone de l'extrémité distale de celui-ci et partiellement en sortant à l'extrémité proximale de l'instrument à l'extérieur de l'instrument, en arrivant d'un réservoir (16) délivrant un liquide d'irrigation sous pression, ou conduisant à un organe de rejet, ledit dispositif comportant également un dispositif de régulation de la pression dans la vessie, lequel présente un régulateur (18) de pression qui est disposé à un niveau fixe dans un canal de mesure formant le conduit d'alimentation (17a, 17b, 10) ainsi qu'une valve (31, 32) qui fait varier la section transversale de l'écoulement et qui est commandée par un capteur de pression qui établit, du côté de la valve orienté en direction de la vessie, quelle est la pression dans le conduit d'alimentation (17b, 10), caractérisé en ce que le capteur de pression est constitué par une membrane (27) chargée par un ressort et sollicitée d'un côté par le liquide d'irrigation, et de l'autre côté par l'air ambiant, laquelle membrane est disposée à un niveau fixe et dont la déviation actionne mécaniquement la soupape (30) de la valve.

2. Dispositif selon la revendication 1, caractérisé en ce que le régulateur (18) de pression est disposé dans la partie (17a, 17b) du conduit d'alimentation (17a, 17b, 10) située à l'extérieur de l'instrument.

3. Dispositif selon la revendication 1, caractérisé en ce qu'une queue (29) de la soupape (3) de valve est fixée au côté de la membrane (27) orienté vers le liquide d'irrigation et en ce qu'un orifice (31) de valve, s'étendant sensiblement perpendiculairement à la membrane, traverse une cloison (24) s'étendant sensiblement parallèlement à la membrane, sur le côté arrière de laquelle la cloison supporte un plateau (33) de soupape, la cloison étant prévue entre une chambre raccordée au réservoir (16) et une chambre raccordée à la vessie (1) et disposée en direction de la membrane (27).

4. Dispositif selon la revendication 3, caractérisé en ce que l'orifice (31) de valve a une forme circulaire, en ce que la surface du plateau (33) de soupape orientée vers la membrane (27) a une forme qui s'effile coniquement, la section transversale la plus étroite de la surface conique étant de plus faible dimension que l'orifice (31) de valve et sa section transversale la plus large étant de plus grande dimension que l'orifice (31) de valve.

5. Dispositif selon l'une des revendications 1 à 4, caractérisé en ce que, du côté de l'air, la membrane (27) est soutenue par un ressort (35).

6. Dispositif selon la revendication 5, caractérisé en ce que la force du ressort (35) peut être réglée au moyen d'un dispositif de réglage réglable depuis l'extérieur.

7. Dispositif selon l'une des revendications 1 à 6, caractérisé en ce qu'est prévu un dispositif d'actionnement qui, après avoir surmonté un jeu, agit dans le sens de l'ouverture sur la soupape (30) de valve.

8. Dispositif selon l'une des revendications 1 à 7, caractérisé en ce que les pièces (19, 20, 21) formant le corps du régulateur de pression (18) sont en matière plastique.

9. Dispositif selon l'une des revendications précédentes, caractérisé en ce qu'un dispositif de mesure de débit (45) est prévu dans le conduit d'alimentation (17a, 17b, 10) ou dans le conduit d'évacuation (15, 14).
